# EUROPÄISCHE PATENTSCHRIFT

(11) **EP 4 390 428 B1**
(45) Veröffentlichungstag und Bekanntmachung des Hinweises auf die Patenterteilung: **10.12.2025**
(21) Anmeldenummer: 22214872.8
(22) Anmeldetag: 20.12.2022
(51) Int. Cl.: G01R 33/28

(54) **VERFAHREN ZUR POSITIONIERUNG EINER PATIENTENLIEGE IN EINER MAGNETRESONANZEINRICHTUNG, POSITIONSFESTLEGUNGSMITTEL UND MAGNETRESONANZEINRICHTUNG**
METHOD FOR POSITIONING A PATIENT SUPPORT IN A MAGNETIC RESONANCE DEVICE AND MAGNETIC RESONANCE DEVICE
PROCÉDÉ DE POSITIONNEMENT D'UN SUPPORT DE PATIENT DANS UN DISPOSITIF DE RÉSONANCE MAGNÉTIQUE ET DISPOSITIF DE RÉSONANCE MAGNÉTIQUE

(43) Veröffentlichungstag der Anmeldung: 26.06.2024
(73) Patentinhaber: Siemens Healthineers AG, 91301 Forchheim (DE)
(72) Erfinder: Biber, Stephan, 91056 Erlangen (DE)
(74) Vertreter: Siemens Healthineers Patent Attorneys

(56) Entgegenhaltungen:
- EP-A1- 3 400 870
- DE-A1- 102009 021 026
- DE-A1- 102014 202 033
- DE-A1- 102016 203 255
- DE-A1- 102017 202 399

## Beschreibung

Die Erfindung betrifft ein Verfahren zur Positionierung einer Patientenliege in einer Patientenaufnahme einer Magnetresonanzeinrichtung, wobei die insbesondere zylindrische Patientenaufnahme durch eine Hauptmagneteinheit mit einem ein Hauptmagnetfeld erzeugenden Hauptmagneten definiert ist. Daneben betrifft die Erfindung eine Magnetresonanzeinrichtung.

Bei der Magnetresonanzbildgebung werden Kernspins eines zu untersuchenden Objekts, insbesondere eines Patienten, durch ein Hauptmagnetfeld (B0-Feld) ausgerichtet, angeregt und der Zerfall der Anregung vermessen. Dabei definiert sich das Sichtfeld einer Magnetresonanzeinrichtung durch das sogenannte Homogenitätsvolumen, in welchem die Homogenität des Hauptmagnetfeldes bestimmten Anforderungen genügt, die eine hochqualitative Magnetresonanzbildgebung erlauben. Heutige Magnetresonanzeinrichtungen weisen üblicherweise eine Hauptmagneteinheit mit einem superleitenden Hauptmagneten auf, die eine zylindrische Patientenaufnahme definieren, in die der Patient mittels einer Patientenliege eingefahren werden kann. Um ein gutes Bildgebungsergebnis zu erhalten, ist es wesentlich, den Untersuchungsbereich des Patienten möglichst genau im Sichtfeld der Magnetresonanzeinrichtung zu positionieren. Dieses befindet sich üblicherweise mittig in der schwer zugänglichen, zylindrischen Patientenaufnahme. Aufgrund dieser Konstellation wird die Sichtfeldmitte bei einer Magnetresonanzeinrichtung teilweise auch als das Isozentrum der Magnetresonanzeinrichtung bezeichnet und kann mit der Mitte der Patientenaufnahme zusammenfallen.

Bei modernen Magnetresonanzeinrichtungen soll es einem Benutzer auf möglichst einfache Art und Weise, insbesondere unter Vereinfachung des Workflows, möglich sein, den Untersuchungsbereich, gegebenenfalls auch unter Berücksichtigung zu verwendender Lokalspulen/Lokalspulenanordnungen, im Sichtfeld der Magnetresonanzeinrichtung zu positionieren. Hierzu wurden bereits Lösungen vorgeschlagen, um bei noch nicht in die Patientenaufnahme eingefahrener Patientenliege Zielpositionen zu definieren, insbesondere entlang der Längsrichtung der Patientenliege, die auch deren Bewegungsrichtung in die Patientenaufnahme entspricht und bei einer zylindrischen Patientenaufnahme üblicherweise auch der Längsrichtung der zylindrischen Patientenaufnahme entspricht. Diese Richtung wird häufig als die z-Richtung bezeichnet, die hierzu senkrechte horizontale Richtung als die x-Richtung und die zur z-Richtung senkrechte vertikale Richtung als y-Richtung.

Zur Festlegung einer Längsposition (z-Position) der Patientenliege, die im Sichtfeld, insbesondere der Sichtfeldmitte, zu positionieren ist, wurde dabei zum einen vorgeschlagen, ein beispielsweise oberhalb der Einfahrt in die Patientenaufnahme vorgesehenes Lasermarkierungsmittel vorzusehen, welches einen Markierungspunkt oder eine Markierungslinie von oben auf die Patientenliege (beziehungsweise den Patienten/auf diesem angeordnete Lokalspulenanordnungen) projiziert. Hiermit kann insbesondere eine Position auf dem Patienten festgelegt werden, die vom Bedienpersonal visuell nachvollzogen werden kann. Nachdem die Liegenposition und die Position des Sichtfelds einer Steuereinrichtung der Magnetresonanzeinrichtung bekannt sind, kann ein entsprechender Versatz zum Sichtfeld ermittelt werden, um den die Patientenliege bewegt wird, um den Patienten derart in die Patientenaufnahme einzufahren, dass die mit der Lasermarkierungseinrichtung markierte Position zumindest in Längsrichtung mit der Sichtfeldmitte übereinstimmt. Allerdings erweist sich die Bedienung der Lasermarkierungseinrichtung vor allem dahingehend als schwierig, dass der Patient letztlich durch Bewegen der Patientenliege geeignet zur Markierung platziert werden muss und sodann noch eine Bestätigung stattfinden muss.

In einem anderen Ansatz wurde vorgeschlagen, entlang des Randes der Patientenliege Markierungen und/oder Bedienmittel vorzusehen, sodass über Eingabe der entsprechenden Markierung beziehungsweise Betätigung des entsprechenden Bedienmittels ebenso eine Längsposition entlang der Patientenliege ausgewählt werden kann, die im Sichtfeld, insbesondere der Sichtfeldmitte, zu positionieren ist. Damit ist allerdings lediglich eine äußerst grobe Positionsangabe möglich. Dies steht in Widerspruch zu hochgenauen Betriebsmöglichkeiten von Motoren in Magnetfeldern. Beispielsweise wird in DE 10 2020 211 327 A1 eine Liege mit einem DC-Motor vorgeschlagen, in dem, beispielsweise mittels eines Hall-Sensors, eine Winkelstellung von Wicklungen des Motors zum Hauptmagnetfeld gemessen werden soll, um eine geeignete Wicklung auszuwählen. DE 10 2017 202 399 A1 beschreibt ein Verfahren und eine Vorrichtung zur Positionierung in einem Magnetfeld eines Magnetresonanztomographen, worin die Liege eine Mehrzahl von Magnetfeldstärkesensoren aufweisen kann, die beabstandet in deren Längsrichtung beziehungsweise Bewegungsrichtung vorgesehen sind.

DE 10 2016 203 255 A1 offenbart ein Verfahren und eine Vorrichtung zur Positionsbestimmung in einem Magnetresonanztomographen, bei dem vorgeschlagen wird, eine äußerst genaue **z-**Referenz beim Einfahren der beweglichen Vorrichtung in die Patientenaufnahme dadurch zu ermitteln, dass ein sogenannter "sweet spot" detektiert wird, an welchem für eine Vielzahl an x-y-Koordinatenpaaren die Magnetfeldstärke einen im Wesentlichen gleichen Wert bei einem gleichen z-Koordinatenwert annimmt. Daher wird ein Magnetfeldstärkesensor in der beweglichen Vorrichtung angeordnet, mithilfe dessen überprüft wird, ob die charakteristische Magnetfeldstärke an diesem Punkt erreicht ist. So kann auf einfache Weise ein Referenzwert für die z-Koordinate festgelegt und nachfolgende Bewegungen darauf bezogen werden.

DE 10 2017 202 399 A1 beschreibt ein Verfahren zur Positionierung einer Patientenliege in einem statischen Magnetfeld eines MR-Tomographen mittels einer Mehrzahl von Magnetfeldstärkesensoren an der Positioniervorrichtung.

Unabhängig vom grammatikalischen Geschlecht eines bestimmten Begriffes sind Personen mit männlicher, weiblicher oder anderer Geschlechteridentität mit umfasst.

Der Erfindung liegt die Aufgabe zugrunde, ein Workflows vereinfachendes und doch hinreichend genaues Verfahren und Hilfsmittel zur Festlegung einer Zielposition für die Patientenliege zumindest in deren Längsrichtung anzugeben.

Zur Lösung dieser Aufgabe ist bei einem Verfahren der eingangs genannten Art erfindungsgemäß vorgesehen, dass
- ein einen, insbesondere dreidimensionalen, Magnetfeldsensor umfassendes Positionsfestlegungsmittel auf der Patientenliege oder einem auf der Patientenliege gelagerten Patienten an einer Markerposition positioniert wird,
- die Sensordaten des Magnetfeldsensors zur Ermittlung der Markerposition in einem Koordinatensystem der Magnetresonanzeinrichtung, in dem auch eine Sichtfeldposition eines Sichtfelds der Magnetresonanzeinrichtung bekannt ist, mittels einer Steuereinrichtung ausgewertet werden,
- durch die Steuereinrichtung aus der Markerposition zumindest in einer einer Bewegungsrichtung der Patientenliege entsprechenden Längsrichtung der Patientenliege eine gewünschte Sollposition des Sichtfelds relativ zu der Markerposition für eine nachfolgende Messung von Magnetresonanzdaten ermittelt wird, und
- durch die Steuereinrichtung unter Verwendung der Markerposition und der Sichtfeldposition die Patientenliege derart angesteuert wird, dass die gewünschte relative Sollposition des Sichtfelds zu der Markerposition eingestellt wird.

Die Längsrichtung der Patientenliege und somit eine Bewegungsrichtung, in der die Patientenliege verschiebbar ist, kann hierbei insbesondere der Längsrichtung der insbesondere zylindrischen Patientenaufnahme entsprechen, mithin die oft auch so bezeichnete z-Richtung der Magnetresonanzeinrichtung darstellen. Als x-Richtung kann die hierzu senkrechte horizontale Richtung angesehen werden, als y-Richtung die zur **z-**Richtung senkrechte vertikale Richtung. In einem beispielsweise derart definierten Koordinatensystem der Magnetresonanzeinrichtung ist die Sichtfeldposition üblicherweise bekannt. Beispielsweise kann es denkbar sein, als Ursprung des Koordinatensystems die Sichtfeldmitte anzusetzen, welche mit besonderem Vorteil der Mitte der Patientenaufnahme entsprechen kann. In diesem Koordinatensystem kann auch eine aktuelle Liegenposition der Patientenliege bestimmt werden. Erfindungsgemäß wird nun vorgeschlagen, ein insbesondere kompaktes, einfach handhabbares Positionsfestlegungsmittel zu verwenden, welches zum einen in der Lage ist, optisch durch seine Anordnung für eine Bedienperson eine gewählte Markerposition zu beschreiben, zum anderen aber erlaubt, mittels eines darin vorgesehenen Magnetfeldsensors, insbesondere Magnetfeldstärkesensors, diese Markerposition zumindest in z-Richtung und zumindest außerhalb der Patientenaufnahme, insbesondere also im Streufeld des Hauptmagnetfeldes, hinreichend genau in zumindest der Längsrichtung im Koordinatensystem der Magnetresonanzeinrichtung zu bestimmen.

Hierzu misst der Magnetfeldsensor also die Magnetfeldstärke, und zwar insbesondere als einen dreidimensionalen Magnetfeldvektor, wobei es auch denkbar sein kann, einen Betrag des dreidimensionalen Magnetfeldvektors als Magnetfeldstärke zu vermessen. Liegt nun eine Hauptmagnetfeldkarte, die zumindest das Streufeld außerhalb der Patientenaufnahme beschreibt, beziehungsweise also eine Streufeldkarte vor, kann aufgrund der gemessenen Magnetfeldstärke zumindest in z-Richtung auch die Position des Magnetfeldsensors ermittelt werden. Hierbei liegt die Hauptmagnetfeldkarte, beschreibend das Streufeld außerhalb der Patientenaufnahme, im Koordinatensystem der Magnetresonanzeinrichtung vor, sodass durch Abgleich der Sensordaten des Magnetfeldsensors mit der Hauptmagnetfeldkarte eine hochgenaue Positionsbestimmung möglich ist, insbesondere, wenn der dreidimensionale Magnetfeldvektor geliefert wird, mithin auch eine Richtung des gemessenen Magnetfelds. Wurde also, beispielsweise durch eine Bedienperson, das Positionsfestlegungsmittel durch visuelle Beurteilung positioniert, können die Sensordaten des Magnetfeldsensors, insbesondere unter Verwendung einer Hauptmagnetfeldkarte, ausgewertet werden, um auch elektronisch eine entsprechende Markerposition im Koordinatensystem der Magnetresonanzeinrichtung vorliegen zu haben. Nachdem zudem innerhalb der Steuereinrichtung bekannt ist, wie bezüglich der Markerposition das Sichtfeld zu positionieren ist, insbesondere zumindest in der Längsrichtung (z-Richtung) die Markerposition in die Sichtfeldmitte zu verschieben ist, kann ein Verfahrweg der Patientenliege in der Längsrichtung auf einfache Art und Weise ermittelt werden, um die gewünschte Sollposition des Sichtfelds relativ zu dem Magnetfeldsensor, also der Markerposition, des Positionsfestlegungsmittels herzustellen, indem die Patientenliege entsprechend angesteuert wird. Das Positionsfestlegungsmittel ist also als eine geschickte Kombination von optischem Marker und lokalisierbarem elektronischen Marker zu verstehen und kann daher auch als Positions-Tag beziehungsweise Positionsmarker bezeichnet werden.

Dabei wird ausgenutzt, dass der Patient auf der Patientenliege üblicherweise außerhalb der Patientenaufnahme auf die Messung vorbereitet wird und auch die Festlegung der Messpositionen für Magnetresonanzdaten, die die Patientenliege einnehmen soll, im Workflow üblicherweise in dieser Vorbereitungsphase stattfindet. Dabei befindet sich die Patientenliege mit dem Patienten aber in dem je nach Position stark unterschiedlichen, also räumlich variierenden Streufeld des Hauptmagnetfeldes, welches vermessen werden kann und als Grundlage für eine genaue Positionsbestimmung mittels des Magnetfeldsensors hergenommen werden kann, zumindest solange sich die Patientenliege mit dem Patienten in eben diesem Streufeld befindet.

Das Positionsfestlegungsmittel ist dabei bevorzugt als separate, kleine, gut handhabbare Vorrichtung ausgebildet, die bevorzugt keinem Zweck bei der folgenden Messung der Magnetresonanzdaten dient, insbesondere diese auch nicht stört. Insbesondere kann das Positionsfestlegungsmittel separat zu zur Messung von Magnetresonanzdaten zu verwendenden Lokalspulenanordnungen oder sonstigen, größeren Hilfsmitteln zur Messung ausgebildet sein, um auf möglichst einfache und direkte Weise optisch die Position indizieren zu können und hierbei unabhängig von anderen, gegebenenfalls bestimmte Positionierungsanforderungen aufweisenden oder in der Positionierung eingeschränkten Hilfsmitteln zu sein.

Mit anderen Worten kann das Positionsfestlegungsmittel also als Positions-Tag beziehungsweise Positionsmarker verstanden werden. Das Positionsfestlegungsmittel wird an der Markerposition auf der Patientenliege (beziehungsweise dem darauf angeordneten Patienten) positioniert, die von der Magnetresonanzeinrichtung in das Sichtfeld gefahren werden soll beziehungsweise deren Lage zum Sichtfeld klar definiert ist. Die Magnetresonanzeinrichtung erkennt mittels der Steuereinrichtung die Markerposition des Positionsfestlegungsmittels und kennt daraufhin die Sollposition und den damit geäußerten Wunsch des Benutzers, den Patienten mittels der Patientenliege mit einer entsprechenden Zielkoordinate zum Sichtfeld zu positionieren. Somit ist ein einfach herstellbares, kleines und vielseitiger einsetzbares, bevorzugt drahtloses Positionsfestlegungsmittel gegeben, welches durch Positionieren auf der Patientenliege oder dem Patienten genutzt werden kann, um der Magnetresonanzeinrichtung auf äußerst einfache und komfortable Art und Weise die gewünschte Sollposition mitzuteilen.

Konkret kann in der Steuereinrichtung eine Ermittlungsinformation abgelegt sein oder durch diese ermittelt werden, die beschreibt, wie die relative Sollposition des Sichtfelds zu der Markerposition bestimmt werden soll. Die Ermittlungsinformation kann dabei fest vorgegeben werden, aus einer Benutzereingabe hergeleitet werden und/oder mit besonderem Vorteil durch die Art der Platzierung des Positionsfestlegungsmittels vorgesehen werden. Insbesondere, worauf im Folgenden noch näher eingegangen werden wird, kann zusätzlich zu der Markerposition auch eine die Orientierung des Positionsfestlegungsmittels beschreibende Orientierungsinformation ermittelt werden, aus der die Ermittlungsinformation, beispielsweise mittels einer Zuordnungsvorschrift, hergeleitet werden kann. In einer konkreten, besonders intuitiven Ausgestaltung kann vorgesehen sein, dass durch die Steuereinrichtung die gewünschte Lage des Sichtfelds als die Sichtfeldmitte zumindest in Längsrichtung an der Markerposition, mithin der Position des Positionsfestlegungsmittel/des Magnetfeldsensors, ermittelt wird. Mit anderen Worten kann durch Positionierung des Positionsfestlegungsmittels auf der Patientenliege beziehungsweise dem Patienten zumindest in Längsrichtung unmittelbar definiert werden, wo die Sichtfeldmitte des Sichtfelds der Magnetresonanzeinrichtung zur Messung der Magnetresonanzdaten liegen soll. In diesem Fall gibt eine Ermittlungsinformation also einfach an, dass die Markerposition zumindest bezüglich der Längsrichtung (z-Richtung) in die Sichtfeldmitte verschoben werden soll. Dies ist eine intuitiv für einen Benutzer besonders klar verständliche Variante.

Grundsätzlich sind jedoch auch andere Szenarien denkbar, beispielsweise wenn eine Positionierbarkeit des Positionsfestlegungsmittels exakt an der richtigen Längsposition nicht möglich ist, Aufnahmekonfigurationen gegeben sind, bei denen mehrere Aufnahmeschritte vorliegen, und dergleichen. So kann beispielsweise dann, wenn mehrere Schichtstapel mit in Längsrichtung versetzter Stapelposition aufgenommen werden sollen, vorgesehen sein, dass sich die Markerposition auf die Mitte der Gesamtheit der Schichtstapel bezieht und somit für den ersten Schichtstapel eine in Längsrichtung versetzte Position anzufahren ist, was entsprechend durch die genannte Ermittlungsinformation angegeben werden kann. Ein entsprechender Versatz kann in der Steuereinrichtung schon bekannt sein und die Ermittlungsinformation kann darauf verweisen, diesen Versatz zusätzlich anzuwenden.

In einer besonders bevorzugten Weiterbildung der vorliegenden Erfindung kann vorgesehen sein, dass das Positionsfestlegungsmittel einen an dessen Außenseite vorgesehenen, mit der Position des Magnetfeldsensors in dem Positionsfestlegungsmittel korrespondierenden, optischen Marker zur visuellen Beschreibung der aktuellen Markerposition aufweist. Beispielsweise kann, insbesondere auf allen verschieden orientierten Außenflächen des Positionsfestlegungsmittels, ein Kreuz oder dergleichen vorgesehen sein, welches die Position des Magnetfeldsensors angibt und somit die Position, die als Markerposition gemessen wird. Insbesondere bei ausgedehnteren Positionsfestlegungsmitteln, in denen, wie noch genauer dargelegt werden wird, weitere Komponenten vorgesehen sind und/oder die zur besseren Handhabbarkeit größer gestaltet sind, wird der Benutzer auf diese Art und Weise unmittelbar informiert, wie er eine genaue Positionierung erreichen kann, sodass technisch durch die Steuereinrichtung die gewollte Markerposition ermittelt wird.

Vorzugsweise kann das Positionsfestlegungsmittel ferner eine Energieversorgungseinrichtung und/oder eine drahtlose Kommunikationseinrichtung aufweisen, mittels der die Sensordaten drahtlos an die Steuereinrichtung übermittelt werden. Der Magnetfeldsensor ist also beispielsweise mit einer Energieversorgungseinrichtung und einer drahtlosen Kommunikationseinrichtung verbunden, sodass das Positionsfestlegungsmittel insgesamt drahtlos ausgestaltet werden kann und die Handhabbarkeit nochmals deutlich verbessert werden kann. Insbesondere ist ein Kommunikationsbetrieb während der Messung der Magnetresonanzdaten, wenn sich also das Positionsfestlegungsmittel innerhalb der Magnetresonanzaufnahme befindet, weder notwendig noch aktiviert, da innerhalb des homogenen Hauptmagnetfeldes im Sichtfeld ohnehin keine sinnvolle Ortsbestimmung möglich wäre. Mithin kann es durch die Kommunikationseinrichtung auf diese Weise zu keiner Störung kommen. Bei der Energieversorgungseinrichtung kann es sich beispielsweise um eine Batterie, insbesondere ein Akkumulator, und/oder einen SuperCap handeln. Hierbei kann insbesondere vorgesehen sein, dass die Energieversorgungseinrichtung durch bei einer Messung von Magnetresonanzdaten auftretende Wechselfelder in der Patientenaufnahme aufgeladen wird. Mit anderen Worten kann also die Energieversorgungseinrichtung zum Energie-Harvesting aus wechselnden Magnetfeldern, insbesondere dem Hochfrequenz- und/oder dem Gradientenfeld, während der Messung der Magnetresonanzdaten ausgebildet sein. Dies ist insbesondere dann zweckmäßig, wenn bei der Messung das auf der Patientenliege beziehungsweise dem Patienten verbliebene Positionsfestlegungsmittel nicht innerhalb des Sichtfelds befindlich ist und somit die Messung auch nicht stört. Soll beispielsweise die Sollposition ohnehin nur in Längsrichtung festgelegt werden, kann es ausreichend sein, das Positionsfestlegungsmittel am Rand der Patientenliege zu positionieren, mithin abseits des Sichtfelds, sodass ohne Störung des Aufnahmebetriebs ein Aufladen der Energieversorgungseinrichtung aus den bei der Messung angewandten Wechselfeldern erfolgen kann. Insbesondere dann, wenn das Positionsfestlegungsmittel während der Messung der Magnetresonanzdaten innerhalb des Sichtfelds angeordnet ist, können auch bereits vorgeschlagene Techniken, die leicht gegenüber der Larmorfrequenz (Magnetresonanzfrequenz) versetzte Frequenzen einsetzen, um Energie zu übertragen, angewendet werden. Denkbar ist es im Rahmen der vorliegenden Erfindung jedoch selbstverständlich auch, die Energieversorgungseinrichtung auf herkömmliche Art und Weise zu laden, beispielsweise mittels einer entsprechend vorgesehenen Ladeschale, in der das Positionsfestlegungsmittel angeordnet werden kann, wenn es nicht benötigt wird.

Die Kommunikationseinrichtung verbindet den Magnetfeldsensor mit einer entsprechenden drahtlosen Kommunikationseinrichtung der Magnetresonanzeinrichtung, insbesondere der Hauptmagneteinheit und/oder einer anderen, insbesondere außerhalb der Patientenaufnahme angeordneten Komponente der Magnetresonanzeinrichtung. Beispielsweise kann die weitere Kommunikationseinrichtung in einem Fuß, der gemeinsam mit der Patientenliege einen Patiententisch bildet, vorgesehen werden. Hierbei können bevorzugt einfache, im Stand der Technik bereits verfügbare Kommunikationstechniken und Kommunikationsprotokolle zur drahtlosen Kommunikation des Positionsfestlegungsmittels mit der Steuereinrichtung eingesetzt werden. Beispielsweise kann die Kommunikation über Bluetooth, ZigBee, WiFi, WiGig, MedRadio und dergleichen erfolgen. Insbesondere ohnehin medizinisch eingesetzte Kommunikationstechniken und Kommunikationsprotokolle, wie beispielsweise MedRadio, können somit vorteilhaft einer weiteren Anwendung zugeführt werden.

Vorzugsweise können die Sensordaten auf Anfrage und/oder in regelmäßigen und/oder, insbesondere bei Verwendung mehrerer Positionsfestlegungsmittel, zufällig gewählten Zeitabständen automatisch übertragen werden. Das Positionsfestlegungsmittel kann, wenn es eingeschaltet ist, mithin in regelmäßigen Abständen (pull) oder auf Anfrage (push) die vom Magnetfeldsensor aufgenommenen Sensordaten an die Steuereinrichtung übermitteln. Insbesondere jedoch dann, wenn, beispielsweise für eine mehrschrittige Untersuchung eines Patienten, mehrere Positionsfestlegungsmittel verwendet werden, beispielsweise zur Definition aufeinanderfolgend anzufahrender Sollpositionen, kann es zweckmäßig sein, die Zeitintervalle zwischen Übermittlungen der Sensordaten zufällig zu wählen, insbesondere als regelmäßige Übertragung bei zufällig gewählter Zeitintervalllänge und/oder insgesamt zufälliger Zeitintervalllänge. Auch ein zufälliger Versatz kann genutzt werden. Auf diese Weise wird die Wahrscheinlichkeit minimiert, dass sich die Übermittlungen einzelner Positionsfestlegungsmittel überschneiden beziehungsweise stören.

Dabei sei an dieser Stelle noch angemerkt, dass bevorzugt die rohen, gegebenenfalls kalibrierten, Sensordaten zur weiteren Auswertung an die Steuereinrichtung übertragen werden, es in Ausführungsbeispielen jedoch durchaus denkbar ist, eine Vorauswertung seitens des Positionsfestlegungsmittels, insbesondere des Magnetfeldsensors selber, vorzunehmen. Insbesondere werden bei der Auswertung der Sensordaten zur Ermittlung der Markerposition zumindest eine Koordinate in Längsrichtung (z-Koordinate), gegebenenfalls jedoch auch weitere räumliche Koordinaten, insbesondere eine x-Koordinate und eine y-Koordinate, und/oder eine Orientierungsinformation ermittelt, worauf im Folgenden noch näher eingegangen werden wird.

Als Magnetfeldsensor kann bevorzugt ein Hall-Sensor verwendet werden und/oder dem Magnetfeldsensor kann ein Orientierungssensor, insbesondere ein Beschleunigungssensor, zugeordnet sein. Dabei handelt es sich bei dem Magnetfeldsensor mit besonderem Vorteil um einen dreidimensionalen Hall-Sensor, der einen dreidimensionalen Magnetfeldvektor an seiner Position bereitstellen kann. Grundsätzlich denkbar ist es jedoch auch, insbesondere dann, wenn aufgrund des Verlaufs des Streufelds des Grundmagnetfelds keine eindeutige Zuordnung aufgrund einer Hauptmagnetfeldkarte möglich ist, dem Magnetfeldsensor zusätzlich einen Orientierungssensor, beispielsweise einen Beschleunigungssensor beziehungsweise Neigungssensor, zuzuordnen.

Eine zweckmäßige Weiterbildung der vorliegenden Erfindung sieht vor, dass bei Überschreiten eines einen Eintritt in das Streufeld der Magnetresonanzeinrichtung anzeigenden Schwellwerts durch das gemessene Magnetfeld, insbesondere die Magnetfeldstärke, des insbesondere in einem Standby-Modus betriebenen Magnetfeldsensors und/oder aufgrund von eine Bewegung anzeigenden Messdaten eines Bewegungssensors des Positionsfestlegungsmittels automatisch das gesamte Positionsfestlegungsmittel aktiviert wird, insbesondere der Magnetfeldsensor in einen Vollbetriebsmodus geschaltet und die Kommunikationseinrichtung aktiviert wird. Auf diese Weise muss das Positionsfestlegungsmittel nicht mit einem Anschaltknopf oder dergleichen versehen werden beziehungsweise von extern über ein Anschaltsignal aktiviert werden, sondern es kann innerhalb des Positionsfestlegungsmittels selbst festgestellt werden, ob es sozusagen in eine Betriebsposition verbracht wird, das bedeutet, auf der Patientenliege und/oder dem Patienten positioniert wird. Beispielsweise kann also der Magnetfeldsensor das Positionsfestlegungsmittel aktivieren und somit einschalten, sobald er in das Streufeld des Hauptmagnetfeldes eingebracht wird, was durch einen geeigneten Schwellwert für die Magnetfeldstärke beschrieben werden kann. Hierbei kann sich der Magnetfeldsensor zunächst in einem energiesparenden Standby-Modus befinden, in dem insbesondere lediglich überwacht wird, ob die gemessene Magnetfeldstärke den Schwellwerte überschreitet. Ist dies der Fall, wird der Magnetfeldsensor in einen Vollbetriebsmodus geschaltet und weitere Komponenten, insbesondere die Kommunikationseinrichtung, werden aktiviert, sodass die Sensordaten an die Steuereinrichtung übertragen werden können. Zusätzlich oder alternativ kann das Positionsfestlegungsmittel auch einen Bewegungssensor umfassen, sodass das Positionsfestlegungsmittel bei insbesondere hinreichend starker Bewegung in einen aktiven Zustand gebracht, insbesondere eingeschaltet, werden kann. Vorzugsweise wird bei Auslösen durch einen Bewegungssensor das Positionsfestlegungsmittel, insbesondere auch dessen Kommunikationseinrichtung, für einen bestimmten Zeitraum, beispielsweise 1 bis 10 Minuten, auch dann weiterbetrieben, wenn keine weiteren starken Bewegungen folgen, da gegebenenfalls kleine Nachjustierungen erfolgen, bevor die Messung der Magnetresonanzdaten dann tatsächlich gestartet wird.

In einer besonders bevorzugten Weiterbildung kann vorgesehen sein, dass durch die Steuereinrichtung unter Verwendung der Sensordaten des Magnetfeldsensors und/oder des Orientierungssensors auch eine die Orientierung des Positionsfestlegungsmittels beschreibende Orientierungsinformation bestimmt wird und die Steuereinrichtung aus der Orientierungsinformation mittels einer Orientierungen zu Steuerinformationen, die eine nachfolgende Bewegung der Patientenliege und/oder eine nachfolgende Messung von Magnetresonanzdaten betreffen, und/oder Ermittlungsinformationen, die die Ermittlung der Sollposition aus der Markerposition beschreiben, zuordnenden Zuordnungsvorschrift eine Steuerinformation und/oder eine Ermittlungsinformation bestimmt, wobei die Steuereinrichtung die Magnetresonanzeinrichtung gemäß der Steuerinformation steuert und/oder die Sollposition gemäß der Ermittlungsinformation ermittelt. Mit anderen Worten kann der Benutzer das Positionsfestlegungsmittel nutzen, um der Steuereinrichtung weitere Informationen mitzuteilen, indem den Orientierungen des Positionsfestlegungsmittels semantische Bedeutungen mittels einer Zuordnungsvorschrift zugeordnet werden und eine entsprechende, die Orientierung beschreibende Orientierungsinformation ermittelt wird. Weist das Positionsfestlegungsmittel beispielsweise ein mehrere Auflageflächen bereitstellendes Gehäuse auf, beispielsweise ein quaderförmiges Gehäuse, kann ein Auflegen dieses Gehäuses mit einer ersten Seite nach oben oder einer ersten Seite nach unten eine andere Bedeutung haben als das Auflegen mit einer zweiten Seite nach oben oder nach unten. Die entsprechende Zuordnung von Steuerinformationen und/oder Ermittlungsinformationen zu Orientierungen kann hierbei wenigstens teilweise fest vorgegeben sein und/oder wenigstens teilweise durch einen Benutzer festlegbar sein. Nimmt man bei einem quaderförmigen Gehäuse neben den sechs Seiten noch eine Orientierung in 90°-Schritten hinzu, können sogar 24 Bedeutungen kodiert werden. Neben der Quaderform sind selbstverständlich auch andere Formen möglich, beispielsweise auch Dodekaederformen und dergleichen. In diesem Zusammenhang ist es besonders vorteilhaft, wenn das Positionsfestlegungsmittel an dessen Außenseite vorgesehene, optische Markierungen zur visuellen Unterscheidung und/oder Zuordnung der Orientierungen umfasst. Diese können insbesondere zusätzlich zu dem die Position des Magnetfeldsensors innerhalb des Positionsfestlegungsmittels anzeigenden optischen Marker vorgesehen sein. Sind beispielsweise bei einem quaderförmigen Positionsfestlegungsmittel nicht nur die Seiten, die nach oben zeigen, relevant, sondern sollen auch Orientierungen der Seiten in der Horizontalen unterschieden werden, ist es denkbar, die gesamte Seite/Oberfläche mit einem die Position des Magnetfeldsensors am Kreuzungspunkt anzeigenden "x" zu markieren, wozu das "x" gleichzeitig Teilflächen definiert, in denen unterscheidbare visuelle Kennzeichnungen, also optische Markierungen, für die Orientierungen vorgesehen werden können. Doch auch, wenn es nur um die Seiten selbst geht, können Markierungen, gegebenenfalls zusätzlich zu dem optischen Marker, vorgesehen werden. Die optischen Markierungen können hierbei abstrakt sein. Möglich ist es jedoch auch, semantische optische Markierungen zu verwenden, beispielsweise Icons, die eine entsprechende mittels der Zuordnungsvorschrift zugeordnete Bedeutung anzeigen.

Konkret können die Steuerinformationen eine Lagerung des Patienten und/oder eine Fahrgeschwindigkeit der Patientenliege und/oder eine zu verwendende Lokalspulenanordnung und/oder ein zu verwendendes Aufnahmeprogramm und/oder eine zu verwendende Magnetresonanzsequenz betreffen. Beispielsweise kann mittels der Orientierung also eine Geschwindigkeit des Einfahrens der Patientenliege in die Patientenaufnahme eingestellt werden, beispielsweise als langsam, schnell oder normal. Auch kann der Magnetresonanzeinrichtung eine Information über die Orientierung des Patienten zugeführt werden, beispielsweise "Patient liegt mit dem Kopf voran oder mit den Füßen voran". Auch Informationen hinsichtlich der Messung der Magnetresonanzdaten selbst können so weitergegeben beziehungsweise eingestellt werden, beispielsweise ein aufzunehmendes Organ oder eine Art der Messung (Diffusionsbildgebung, normale Bildgebung, etc.). Auch die Information, welche Lokalspulenanordnung beziehungsweise Lokalspule einer Lokalspulenanordnung verwendet werden soll, kann relevant sein und als Steuerinformation mittels der Orientierung weitergegeben werden.

Zweckmäßigerweise kann die Ansteuerung der Patientenliege durch die Steuereinrichtung erst bei Erfüllung einer, insbesondere eine Bestätigung durch einen Benutzer anzeigenden, Triggerbedingung erfolgen. Das bedeutet, die Bewegung der Patientenliege und damit des Patienten in die Patientenaufnahme wird erst bei Eintreten eines Triggerevents, dessen Vorliegen durch die Triggerbedingung überprüft wird, ausgelöst. Auf diese Weise wird die Sicherheit erhöht, insbesondere wenn die Triggerbedingung eine Bestätigung durch einen Benutzer anzeigt. Hierbei kann konkret vorgesehen sein, dass die Triggerbedingung die Bestätigung eines insbesondere redundant ausgeführten Bedienelements an der Hauptmagneteinheit und/oder an dem Positionsfestlegungsmittel auswertet. Dabei kann als Bedienelement ein herkömmliches, beispielsweise an der Hauptmagneteinheit als Touchscreen und/oder Taste vorgesehenes, Bedienelement genutzt werden. Zweckmäßigerweise kann ein Bedienelement jedoch auch an dem Positionsfestlegungsmittel angebracht sein, sodass beispielsweise einfach nur das Positionsfestlegungsmittel positioniert werden muss und dann sofort die Fahrt der Patientenliege in die Patientenaufnahme noch am Positionsfestlegungsmittel gestartet werden kann. Dabei kann das Bedienelement des Positionsfestlegungsmittels beispielsweise einen Knopf und/oder einen Touch-Sensor und/oder einen berührungslose Gesten messenden Sensor umfassen. Insbesondere bei Verwendung eines Touch-Sensors und/oder eines Gesten berührungslos messenden Sensors kann in diesem Zusammenhang auch vorgesehen sein, dass die Betätigung eine Geste, insbesondere ein Wischen, umfasst. Beispielsweise kann somit eine Wischbewegung über das Positionsfestlegungsmittel als Auslösebefehl für die Bewegung verstanden werden und die Patientenliege wird entsprechend so verfahren, dass sich die gewünschte relative Position des Sichtfelds zu dem Positionsfestlegungsmittel, konkret dem Magnetfeldsensor, ergibt. Allgemein gesprochen kann diese Auslösefunktion auch redundant ausgelegt sein, um Fehlfunktionen zu minimieren. Hierbei kann beispielsweise vorgesehen sein, dass die redundante Ausführung zwei einem Bedienelement zugeordnete Schalter umfasst, wobei die Triggerbedingung nur dann erfüllt ist, wenn beide Schalter als betätigt festgestellt werden.

In einer Weiterbildung der Erfindung kann vorgesehen sein, dass mehrere, insbesondere eine Reihenfolge vorgebende und/oder eine zugeordnete Identifikationsinformation an die Steuereinrichtung sendende, Positionsfestlegungsmittel bei mehreren aufeinanderfolgenden Messungen von Magnetresonanzdaten in unterschiedlichen Untersuchungsbereichen verwendet werden. Mithin können auch mehrere Positionsfestlegungsmittel als Positions-Tags, insbesondere in Längsrichtung verteilt, verwendet werden, um mehrere unterschiedliche Untersuchungsbereiche anzuzeigen. Zweckmäßigerweise besitzen die Positionsfestlegungsmittel dann eine eindeutig zugeordnete Identifikationsinformation, damit die Steuereinrichtung sie unterscheiden kann. Die Positionsfestlegungsmittel können beispielsweise, auch von außen durch einen Benutzer erkennbar, nummeriert sein, sodass die Untersuchungsbereiche in der entsprechenden Reihenfolge der Positionsfestlegungsmittel angefahren werden können. Wie bereits hinsichtlich der Kommunikation genauer erläutert wurde, kann eine einfache Minimierung einer Störwahrscheinlichkeit bei der Kommunikation dadurch erreicht werden, dass Sendevorgänge zufallsbasiert zeitlich stattfinden. Dies vermeidet eine komplizierte Auslegung der Positionsfestlegungsmittel, indem ein einfaches, zufallsbasiertes Zeitmultiplex eingeführt wird statt eines komplexer umzusetzenden Frequenzmultiplexing oder dergleichen.

Wie bereits erwähnt, kann das Positionsfestlegungsmittel ein, insbesondere quaderförmiges, Gehäuse umfassen, in dem der Magnetfeldsensor aufgenommen ist. Das Gehäuse kann mithin auch die Kommunikationseinrichtung und die Energieversorgungseinrichtung aufnehmen. Das Gehäuse kann beispielsweise aus Kunststoff bestehen, aber auch ein eine gewisse Flexibilität aufweisendes Material umfassen, wie es beispielsweise im medizinischen Bereich auch für Bandagen und dergleichen eingesetzt wird.

Das Positionsfestlegungsmittel kann beispielsweise eine maximale Ausdehnung von 1 bis 10 cm und/oder ein Volumen von 1 bis 100 cm³ aufweisen. Magnetfeldsensoren, beispielsweise dreidimensionale Hall-Sensoren, können inzwischen auf Chip-Größe bereitgestellt werden, sodass auch eine sehr kleine Auslegung des Positionsfestlegungsmittels möglich ist. Beispielsweise kann eine quaderförmige Ausgestaltung mit 2 cm x 2 cm x 10 cm möglich sein. Denkbar sind jedoch auch würfelförmige Ausgestaltungen, beispielsweise mit einer Seitenlänge von 3 cm bis 4,5 cm.

Vorzugsweise kann das Positionsfestlegungsmittel auch Befestigungsmittel aufweisen und mittels des Befestigungsmittels an der Patientenliege und/oder einem Kleidungsstück und/oder einer Decke und/oder einer Lokalspulenanordnung befestigt werden. Beispielsweise kann das Befestigungsmittel als ein Clipmittel und/oder ein Klettmittel ausgebildet sein. Soll das Positionsfestlegungsmittel ohnehin lediglich die Längsposition festlegen, können die Befestigungsmittel beispielsweise als Rastmittel, insbesondere Clipmittel, derart ausgestaltet sein, dass das Positionsfestlegungsmittel seitlich an der Patientenliege befestigt werden kann, beispielsweise in einer seitlich entlang der Längsrichtung verlaufenden Nut der Patientenliege. Möglich ist es auch, dass das Positionsfestlegungsmittel an dem Patienten, insbesondere an Kleidung des Patienten, und/oder an Lokalspulenanordnungen befestigbar ist. Hierbei kann sich insbesondere die Verwendung von Klettmaterialien anbieten, da beispielsweise bei Verwendung eines Hakenmaterials seitens des Befestigungsmittels des Positionsfestlegungsmittels mit einer Vielzahl von beispielsweise Schlaufen aufweisenden Stoffen, insbesondere auch Kleidungsstücken, interagiert werden kann. Auch flexibles Material, das in Lokalspulenanordnungen, die auf den Patienten aufgelegt werden können, verwendet wird, kann geeignet sein, um Klettverbindungen zu tragen. Beispielsweise kann ein Gehäuse des Positionsfestlegungsmittels also als Klettwürfel beziehungsweise allgemein Klettquader ausgebildet sein.

Insbesondere weist das Positionsfestlegungsmittel den Magnetfeldsensor, die Energieversorgungseinrichtung und die Kommunikationseinrichtung auf, bevorzugt in einem, gegebenenfalls auch zumindest teilweise flexiblen, Gehäuse. Es dient bevorzugt keinem Zweck bei der Aufnahme der Magnetresonanzdaten selber, sondern lediglich als Positionierungs-Tag, mit dem ein Benutzer auf besonders einfache Art und Weise eine Markerposition und somit Soll-Position des Patienten auf der Patientenliege relativ zu dem Sichtfeld festlegen kann. Beispielsweise kann das Positionsfestlegungsmittel eine maximale Ausdehnung von 1 bis 10 cm oder ein Volumen von 1 bis 100 cm³ aufweisen. Es kann zudem Befestigungsmittel umfassen, mit denen es an der Patientenliege und/oder einem Kleidungsstück und/oder einer Decke und/oder einer Lokalspulenanordnung befestigt wird. Auch können entsprechende optische Marker und optische Markierungen auf den Oberflächen angeordnet sein, um klar anzuzeigen, wo innerhalb des Positionsfestlegungsmittel der Magnetfeldsensor angeordnet ist und/oder welche gegebenenfalls eine Steuerinformation und/oder eine Ermittlungsinformation anzeigende Orientierung verwendet wird.

Schließlich betrifft die Erfindung auch eine Magnetresonanzeinrichtung, aufweisend eine Hauptmagneteinheit mit einer insbesondere zylindrischen Patientenaufnahme und einem ein Hauptmagnetfeld erzeugenden Hauptmagneten, eine in die Patientenaufnahme entlang einer Längsrichtung einfahrbare Patientenliege, eine Steuereinrichtung und ein auf der Patientenliege und/oder einem auf der Patientenliege gelagerten Patienten frei an einer Markerposition positionierbares Positionsfestlegungsmittel mit einem Magnetfeldsensor und einer Kommunikationseinrichtung zur Übertragung von Sensordaten des Magnetfeldsensors an die Steuereinrichtung, wobei die Steuereinrichtung aufweist:
- eine Auswertungseinheit zur Auswertung der Sensordaten des Magnetfeldsensors zur Ermittlung der Markerposition in einem Koordinatensystem der Magnetresonanzeinrichtung, in dem auch eine Sichtfeldposition eines Sichtfelds der Magnetresonanzeinrichtung bekannt ist,
- eine Ermittlungseinheit zur Ermittlung einer gewünschten Sollposition des Sichtfelds relativ zu der Markerposition aus der Markerposition zumindest in der Längsrichtung der Patientenliege für eine nachfolgende Messung von Magnetresonanzdaten, und
- eine Steuereinheit zu Ansteuerung der Patientenliege unter Verwendung der Markerposition derart, dass die gewünschte relative Sollposition des Sichtfelds zu der Markerposition eingenommen wird.

Auch bezüglich der Magnetresonanzeinrichtung gelten die bisherigen Ausführungen zum erfindungsgemäßen Verfahren und zum Positionsfestlegungsmittel fort.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipskizze einer erfindungsgemäßen Magnetresonanzeinrichtung,
- Fig. 2: den funktionalen Aufbau eines Positionsfestlegungsmittels,
- Fig. 3: eine Aufsicht auf das Positionsfestlegungsmittel,
- Fig. 4: eine Aufsicht auf ein weiteres Ausführungsbeispiel eines Positionsfestlegungsmittels, und
- Fig. 5: einen Ablaufplan eines Ausführungsbeispiels des erfindungsgemäßen Verfahrens.

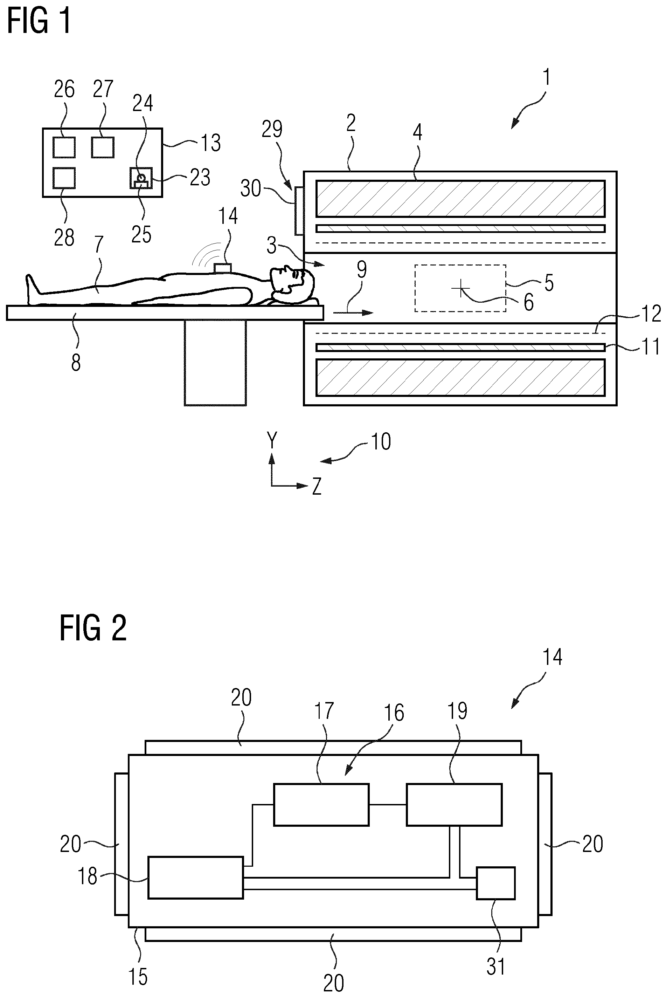

Fig. 1 ist eine Prinzipskizze einer erfindungsgemäßen Magnetresonanzeinrichtung 1. Die Magnetresonanzeinrichtung 1 weist, wie grundsätzlich bekannt, eine Hauptmagneteinheit 2 auf, die vorliegend eine zylindrische Patientenaufnahme 3 definiert. Die Hauptmagneteinheit 2 weist einen hier supraleitenden Hauptmagneten 4 auf, der ein Hauptmagnetfeld der Magnetresonanzeinrichtung 1 erzeugt. Innerhalb der Patientenaufnahme 3 bildet das Hauptmagnetfeld das sogenannte Homogenitätsvolumen als Sichtfeld 5 mit der Sichtfeldmitte 6, die vorliegend auch die Mitte der Patientenaufnahme 3 bildet.

Ein durch Magnetresonanzbildgebung, also Messung von Magnetresonanzdaten, zu untersuchender Patient 7 kann mittels einer Patientenliege 8, die in Fig. 1 außerhalb der Patientenaufnahme 3 gezeigt ist, gemäß dem Pfeil 9 entlang ihrer Längsrichtung als Bewegungsrichtung in die Patientenaufnahme 3 eingefahren werden. Die Längsrichtung der Patientenliege 8 entspricht dabei auch der Längsrichtung der zylindrischen Patientenaufnahme 3 und wird im Koordinatensystem 10 der Magnetresonanzeinrichtung 1 üblicherweise als z-Richtung bezeichnet. Zu sehen ist auch die vertikale, zur z-Richtung senkrechte y-Richtung; die x-Richtung verläuft senkrecht zur Bildebene, ist also die zur z-Richtung senkrechte horizontale Richtung.

Um eine Bildaufnahme zu ermöglichen, schließen an den Hauptmagneten 4 in Richtung zur Patientenaufnahme 3 vorliegend noch Gradientenspulen einer Gradientenspulenanordnung 11 an. Optional ist auch eine Ganzkörper-Hochfrequenzspulenanordnung 12 vorgesehen.

Selbstverständlich können für Untersuchungen des Patienten 7 auch hier nicht näher gezeigte Lokalspulenanordnungen eingesetzt werden, die an der Patientenliege fest installiert oder anordenbar sind und/oder auch auf den Patienten aufgelegt werden können.

Der Betrieb der Magnetresonanzeinrichtung 1 wird durch eine Steuereinrichtung 13, die hier nur schematisch und in ihrem funktionalen Aufbau teilweise angedeutet ist, gesteuert.

Um auf möglichst einfache Weise eine Längsposition, also Position in z-Richtung, die im Sichtfeld 5, konkret der Sichtfeldmitte 6, positioniert werden soll, und somit einen Untersuchungsbereich des Patienten 7 festzulegen, ist ein Positionsfestlegungsmittel 14 vorgesehen, welches auf dem Patienten 7 und/oder der Patientenliege 8 frei positioniert werden kann. Dabei bedeutet eine Positionierung auf dem Patienten 7 vorliegend auch eine Positionierung auf einem auf dem Patienten 7 befindlichen Objekt, beispielsweise einer Lokalspulenanordnung, einer den Patienten abdeckenden Decke und dergleichen.

Fig. 2 zeigt den funktionalen Aufbau des Positionsfestlegungsmittel 14 genauer. Ersichtlich weist dieses in einem Gehäuse 15, vorliegend mittig, einen Magnetfeldsensor 16 auf, welcher als ein dreidimensionaler Hall-Sensor 17 ausgebildet ist. Der dreidimensionale Hall-Sensor 17 misst vorliegend den dreidimensionalen Magnetfeldvektor. In dem Gehäuse sind zudem eine Energieversorgungseinrichtung 18 und eine Kommunikationseinrichtung 19 vorgesehen, mittels derer die Sensordaten des Magnetfeldsensors 16 an die Steuereinrichtung 13 drahtlos übertragen werden können. Dabei können beispielsweise Kommunikationsstandards wie Bluetooth, ZigBee, WiFi, WiGig, MedRadio und dergleichen eingesetzt werden. Die Energieversorgungseinrichtung umfasst insbesondere eine Batterie, beispielsweise einen Akkumulator, und/oder einen SuperCap. In Ausführungsbeispielen kann sie zum Energie-Harvesting bei einer Messung von Magnetresonanzdaten ausgebildet sein, beispielsweise aus Hochfrequenzfeldern und/oder Gradientenfeldern, die bei der Messung auftreten, ist dabei aber idealerweise nicht direkt im Sichtfeld 5 angeordnet, sondern beispielsweise seitlich davon. Ein Aufladen kann jedoch auch mittels einer Ladeschale oder dergleichen vorgenommen werden.

Das Positionsfestlegungsmittel 14 ist vorliegend im Übrigen auch so ausgestaltet, dass es sich automatisch einschaltet, wenn es in Gebrauch genommen wird. Hierzu ist in einem inaktiven ausgeschalteten Zustand nur der Magnetfeldsensor 16 in einem Standby-Modus aktiv und erkennt ein Einbringen in das Streufeld des Hauptmagnetfeldes dadurch, dass ein bestimmter Schwellwert für die Magnetfeldstärke überschritten ist. Wird ein solches Eintreten in das Streufeld des Hauptmagnetfeldes erkannt, wechselt der Magnetfeldsensor 16 in einen Vollbetriebsmodus und aktiviert auch die anderen Komponenten, hier insbesondere die Kommunikationseinrichtung 19.

Die Kommunikationseinrichtung 19 kann grundsätzlich Sensordaten auf Anfrage übertragen, überträgt diese jedoch bevorzugt regelmäßig, beispielsweise in regulären oder aber innerhalb eines erlaubten Intervalls zufällig gewählten Zeitabständen. Die Variante, in der die Zeitabstände zwischen Aussendungen gemäß einer einmalig bestimmten oder einer für jeden Zeitabstand neu bestimmten Zufallszahl gewählt wird, ist insbesondere dann nützlich, wenn auf einfache Art und Weise ohne störenden Funkverkehr mehrere gleich ausgestaltete Positionsfestlegungsmittel 14 eingesetzt werden sollen, da dann die gegenseitige Störwahrscheinlichkeit minimiert wird, ohne dass ein großer Aufwand getrieben werden muss.

Mit den Sensordaten wird auch eine eindeutige Identifikationsinformation des Positionsfestlegungsmittels 14 übersandt, sodass bei einer Verwendung von mehreren Positionsfestlegungsmitteln 14 eine Zuordnung erfolgen kann. Die Identifikationsinformation stellt den einzigen notwendigen Unterschied zwischen den Positionsfestlegungsmitteln 14 dar. Mehrere Positionsfestlegungsmittel 14 können beispielsweise eingesetzt werden, um mehrere, nacheinander aufzunehmende Untersuchungsbereiche zumindest in Längsrichtung zu markieren; in der Steuereinrichtung 13 kann dann bereits eine Reihenfolge anhand der Identifikationsinformation abgelegt sein, die auch außen auf dem Gehäuse 15 erkennbar sein kann.

Das Gehäuse 15 kann aus einem Kunststoff und/oder auch aus einem Gewebe bestehen und/oder wenigstens teilweise flexibel sein, insbesondere, wenn auf dem Patienten 7 gearbeitet werden soll. Es weist Befestigungsmittel 20 an seiner Außenseite auf, die beispielsweise Klettmittel umfassen können, aber auch Clipmittel oder sonstige Rastmittel, beispielsweise zum Verrasten in einer seitlichen Nut der Patientenliege 8.

Das Gehäuse 15 ist vorliegend quaderförmig und kann beispielsweise flach ausgebildet sein, beispielsweise mit den Abmessungen 2 cm x 2 cm x 10 cm. Auch eine würfelförmige Ausgestaltung ist denkbar, beispielsweise mit einer Kantenlänge von 3 cm bis 4,5 cm.

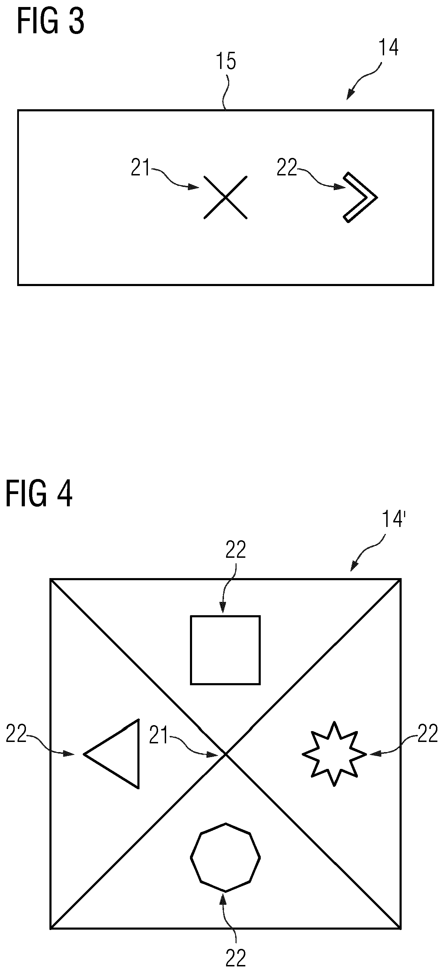

Auf dem Gehäuse 15 sind, wie in Fig. 3 dargestellt, Visualisierungen, die die Verwendung des Positionsfestlegungsmittels 14 vereinfachen, angebracht. So zeigt zunächst ein optischer Marker 21, hier ein Kreuz, an, wo innerhalb des Gehäuses 15 sich der Magnetfeldsensor 16 befindet. Nachdem vorliegend aber auch eine Orientierung des Positionsfestlegungsmittels 16 relevant wird, durch welche ein Benutzer der Steuereinrichtung 13 weitere Informationen vermitteln kann, ist auch eine optische Markierung 22 vorhanden. Im Ausführungsbeispiel der Fig. 3 ist dabei nur relevant, welche Seite des Positionsfestlegungsmittels 14 nach oben weist. Jeder der Seiten kann dann eine Steuerinformation oder eine Ermittlungsinformation zugeordnet werden, wie im Folgenden noch näher erläutert wird. Das vorliegend beispielhaft dargestellte Symbol zeigt einen einzelnen Pfeil und steht für eine langsame Bewegung der Patientenliege 8, wobei beispielsweise zwei Pfeile für eine normale Bewegungsgeschwindigkeit und drei Pfeile für eine schnelle Bewegungsgeschwindigkeit stehen können. Dies ist jedoch als beispielhaft zu verstehen; Steuerinformationen können sich insbesondere auch auf eine Lagerung des Patienten und/oder ein zu verwendendes Aufnahmeprogramm und/oder eine zu verwendende Lokalspulenanordnung und/oder eine zu verwendende Magnetresonanzsequenz beziehen.

Fig. 4 zeigt eine Illustration einer Aufsicht auf ein Positionsfestlegungsmittel 14', bei dem sogar die Ausrichtung der entsprechenden obenliegenden Oberfläche relevant ist, und zwar in 90° Schritten. Hierbei zerteilt der optische Marker 21, der eine mittige Lage des Magnetfeldsensors 16 anzeigt, die sichtbare Oberfläche ohnehin in vier Abschnitte, in denen dann jeweils eine entsprechende optische Markierung 22 vorgesehen wird.

Zurückkehrend zur Fig. 1 zeigt diese schematisch auch den funktionalen Aufbau der Steuereinrichtung 13. Diese weist zunächst ein Speichermittel 23 auf, in dem vorliegend eine Hauptmagnetfeldkarte 24, die insbesondere den räumlichen Verlauf des Streufelds des Hauptmagnetfeldes im Bereich vor der Patientenaufnahme 3, wo sich die Patientenliege 8 in Fig. 1 befindet, beschreibt, und zwar im Magnetresonanzkoordinatensystem 10. Ferner ist dort auch eine Zuordnungsvorschrift 25 abgelegt, die verschiedenen Orientierungen des Positionsfestlegungsmittels 14 Steuerinformationen und/oder Ermittlungsinformationen zuordnet.

In einer Auswertungseinheit 26 der Steuereinrichtung 13 werden die Sensordaten des Magnetfeldsensors 16, die über die Kommunikationseinrichtung 19 übermittelt wurden, ausgewertet, um nicht nur die aktuelle Markerposition des Magnetfeldsensors 16 im Positionsfestlegungsmittel 14 wenigstens in Längsrichtung zu ermitteln, sondern auch eine die Orientierung des Positionsfestlegungsmittels 14 beschreibende Orientierungsinformation. Die Orientierungsinformation wird genutzt, um mittels der Zuordnungsvorschrift 25, insbesondere einer Look-Up-Tabelle, die zugehörige Steuerinformation und/oder die zugehörige Ermittlungsinformation zu bestimmen. Eine Steuerinformation, die beispielsweise eine Geschwindigkeit, mit der die Patientenliege 8 zu bewegen ist, beschreibt, wird dann entsprechend im weiteren Verlauf angewendet.

Die Ermittlungsinformation, sei sie fest vorgegeben, benutzerseitig vorgegeben, oder aus der Orientierungsinformation hergeleitet, beschreibt, welche relative Sollposition zwischen der Markerposition, also dem Magnetfeldsensor 16 beziehungsweise dem Positionsfestlegungsmittel 14, und dem Sichtfeld 5, insbesondere der Sichtfeldmitte 6, hergestellt werden soll. In einer einfachen, bevorzugten und als Vorauswahl gewählten Einstellung soll die Markerposition durch Bewegung der Patientenliege 8 zumindest in Längsrichtung in Übereinstimmung mit der Sichtfeldmitte 6 gebracht werden. Das bedeutet, durch das Positionsfestlegungsmittel 14 wird unmittelbar markiert, was in der Sichtfeldmitte 6 zu positionieren ist.

Es sind jedoch auch abweichende Fälle denkbar, beispielsweise bei einer mehrschrittigen Aufnahme benachbarter Schichtstapel, wo eine Verschiebung für den ersten Schichtstapel berücksichtigt werden kann, wenn das Positionsfestlegungsmittel 14 den Mittelpunkt aller Schichtstapel markieren soll.

Unter Verwendung der Ermittlungsinformation wird diese Soll-position des Sichtfelds 5 relativ zu der Markerposition zumindest in der Längsrichtung der Patientenliege 8 in einer Ermittlungseinheit 27 ermittelt.

Eine Steuereinheit 28 überprüft das Vorliegen einer Triggerbedingung, die anzeigt, dass die Patientenliege jetzt so bewegt werden soll, dass sich die gewünschte relative Sollposition ergibt. Insbesondere wird dann bei Betrachtung der Längsposition, wenn die Markerposition in der Sichtfeldmitte 6 platziert werden soll, die Patientenliege 8 um den Längsrichtungs-Abstand von der Position der Sichtfeldmitte 6, die bekannt ist und insbesondere den Ursprung des Koordinatensystems 10 bildet, und der gemessenen Markerposition verschoben.

Die Triggerbedingung kann dabei beispielsweise überprüfen, ob ein insbesondere redundant ausgeführtes Bedienelement betätigt wurde. Dabei kann es sich um ein Bedienelement 29 an der Hauptmagneteinheit 2 handeln, beispielsweise einen Touchscreen 30, wie dies im Stand der Technik grundsätzlich bekannt ist.

Möglich ist es aber auch, gegebenenfalls zusätzlich, wie in der schematischen Funktionsskizze der Fig. 2 dargestellt, das Positionsfestlegungsmittel 14 mit einem eigenen Bedienelement 31 zu versehen, welches ein Knopf oder ein Touch-Sensor sein kann, vorliegend aber durch einen berührungslosen, Gesten erkennenden Sensor gebildet ist. Wird mittels des Bedienelements 31 eine wischende Bewegung über das Positionsfestlegungsmittel 14 festgestellt, ist die Triggerbedingung erfüllt und die Bewegung kann beginnen. Zur Herstellung der Redundanz kann ein solcher Sensor als Bedienelement 31 mehrfach vorgesehen sein; bei einem zu betätigenden Knopf können auch zwei Schalter durch diesen geschlossen werden, um die Redundanz bereitzustellen.

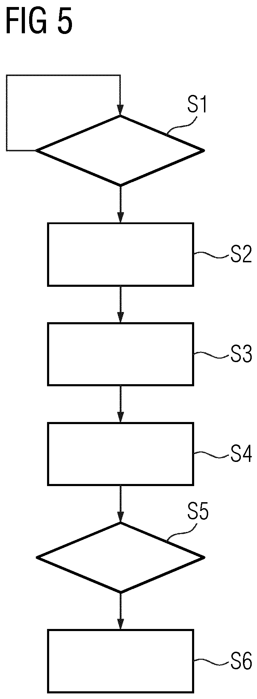

Fig. 5 fasst die Schritte eines erfindungsgemäßen Verfahrens nochmal in einem Ablaufplan zusammen.

In einem Schritt S1 wird bei ausgeschaltetem Positionsfestlegungsmittel 14 durch den Magnetfeldsensor 16 im Standby-Betrieb überwacht, ob das Positionsfestlegungsmittel 14 in das Streufeld des Hauptmagnetfeldes eingebracht wird. Ist dies der Fall, wird das Positionsfestlegungsmittel 14 eingeschaltet. In einem Schritt S2 wird das Positionsfestlegungsmittel 14 an einer gewünschten Markerposition auf der Patientenliege 8 und/oder dem Patienten 7 positioniert. In einem Schritt S3 wird durch die Auswertungseinheit 26 aus den Sensordaten des Magnetfeldsensors 16 durch Abgleich mit der Hauptmagnetfeldkarte 24 die Markerposition im Koordinatensystem 10 der Magnetresonanzeinrichtung 1 ermittelt.

In einem Schritt S4 wird eine gewünschte Sollposition der Markerposition relativ zu der Sichtfeldmitte 6 durch die Ermittlungseinheit 27 ermittelt. In der Steuereinheit 28 wird dann im Schritt S5 das Vorliegen der Triggerbedingung überprüft, ob beispielsweise ein Benutzer über das Positionsfestlegungsmittel 14 gewischt hat. Ist sie erfüllt, kann in einem Schritt S6 die Patientenliege 8 so verfahren werden, dass sich die gewünschte Sollposition zwischen der Markerposition und der Sichtfeldmitte 6 einstellt, insbesondere also der durch das Positionsfestlegungsmittel 14 festgelegte Ort in der Sichtfeldmitte 6 positioniert ist.

Selbstverständlich werden während des Betriebs der Patientenliege 8 und/oder der nachfolgenden Messung von Magnetresonanzdaten auch Steuerinformationen berücksichtigt, falls diese mittels der Zuordnungsvorschrift 25 entsprechend bestimmt wurden.

Es sei an dieser Stelle noch angemerkt, dass statt oder zusätzlich zu dem Standby-Betrieb des Magnetfeldsensors 16 und der Überprüfung gegen wenigstens einen Schwellwert auch ein Bewegungssensor verwendet werden kann, der die Bewegung und Benutzung des Positionsfestlegungsmittels 14 beschreibt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Positionierung einer Patientenliege (8) in einer Patientenaufnahme (3) einer Magnetresonanzeinrichtung (1), wobei die insbesondere zylindrische Patientenaufnahme (3) durch eine Hauptmagneteinheit (2) mit einem ein Hauptmagnetfeld erzeugenden Hauptmagneten (4) definiert ist, **dadurch gekennzeichnet, dass**
- ein einen, insbesondere dreidimensionalen, Magnetfeldsensor (16) umfassendes Positionsfestlegungsmittel (14, 14') auf der Patientenliege (8) oder einem auf der Patientenliege (8) gelagerten Patienten (7) an einer Markerposition positioniert wird,
- die Sensordaten des Magnetfeldsensors (16) zur Ermittlung der Markerposition in einem Koordinatensystem (10) der Magnetresonanzeinrichtung (1), in dem auch eine Sichtfeldposition eines Sichtfelds (5) der Magnetresonanzeinrichtung (1) bekannt ist, mittels einer Steuereinrichtung (13) ausgewertet werden,
- durch die Steuereinrichtung (13) aus der Markerposition zumindest in einer einer Bewegungsrichtung der Patientenliege (8) entsprechenden Längsrichtung der Patientenliege (8) eine gewünschte Sollposition des Sichtfelds (5) relativ zu der Markerposition für eine nachfolgende Messung von Magnetresonanzdaten ermittelt wird, und
- durch die Steuereinrichtung (13) unter Verwendung der Markerposition und der Sichtfeldposition die Patientenliege (8) derart angesteuert wird, dass die gewünschte relative Sollposition des Sichtfelds (5) zu der Markerposition eingenommen wird.

2. Verfahren nach Anspruch 1, **dadurch gekennzeichnet, dass** durch die Steuereinrichtung (13) die gewünschte Lage des Sichtfelds (5) als die Sichtfeldmitte (6) zumindest in Längsrichtung an der Markerposition ermittelt wird.

3. Verfahren nach Anspruch 1 oder 2, **dadurch gekennzeichnet, dass** das Positionsfestlegungsmittel (14, 14') einen an dessen Außenseite vorgesehenen, mit der Position des Magnetfeldsensors (16) in dem Positionsfestlegungsmittel (14, 14') korrespondierenden, optischen Marker (21) zur visuellen Beschreibung der aktuellen Markerposition aufweist.

4. Verfahren nach einem der vorangehenden Ansprüche, **dadurch gekennzeichnet, dass** das Positionsfestlegungsmittel (14, 14') ferner eine Energieversorgungseinrichtung (18) und/oder eine drahtlose Kommunikationseinrichtung (19) aufweist, mittels der die Sensordaten drahtlos an die Steuereinrichtung (13) übermittelt werden.

5. Verfahren nach Anspruch 4, **dadurch gekennzeichnet, dass** die Energieversorgungseinrichtung (18) durch bei einer Messung von Magnetresonanzdaten auftretende Wechselfelder in der Patientenaufnahme (3) aufgeladen wird und/oder die Sensordaten auf Anfrage und/oder in regelmäßigen und/oder, insbesondere bei Verwendung mehrerer Positionsfestlegungsmittel (14, 14'), zufällig gewählten Zeitabständen automatisch übertragen werden.

6. Verfahren nach einem der vorangehenden Ansprüche, **dadurch gekennzeichnet, dass** als der Magnetfeldsensor (16) ein Hall-Sensor (17) verwendet wird und/oder dem Magnetfeldsensor (16) ein Orientierungssensor, insbesondere ein Beschleunigungssensor, zugeordnet ist.

7. Verfahren nach einem der vorangehenden Ansprüche, **dadurch gekennzeichnet, dass** durch die Steuereinrichtung (13) unter Verwendung der Sensordaten des Magnetfeldsensors (16) und/oder des Orientierungssensors auch eine die Orientierung des Positionsfestlegungsmittels (14, 14') beschreibende Orientierungsinformation bestimmt wird und die Steuereinrichtung (13) aus der Orientierungsinformation mittels einer Orientierungen zu Steuerinformationen, die eine nachfolgende Bewegung der Patientenliege (8) und/oder eine nachfolgende Messung von Magnetresonanzdaten betreffen, und/oder Ermittlungsinformationen, die die Ermittlung der Sollposition aus der Markerposition beschreiben, zuordnenden Zuordnungsvorschrift (25) eine Steuerinformation und/oder eine Ermittlungsinformation bestimmt, wobei die Steuereinrichtung (13) die Magnetresonanzeinrichtung (1) gemäß der Steuerinformation steuert und/oder die Sollposition gemäß der Ermittlungsinformation ermittelt.

8. Verfahren nach Anspruch 7, **dadurch gekennzeichnet, dass** die Steuerinformationen eine Lagerung des Patienten (7) und/oder eine Fahrgeschwindigkeit der Patientenliege (8) und/oder eine zu verwendende Lokalspulenanordnung und/oder ein zu verwendendes Aufnahmeprogramm und/oder eine zu verwendende Magnetresonanzsequenz betreffen, und/oder dass das Positionsfestlegungsmittel (14, 14') an dessen Außenseite vorgesehene, optische Markierungen (22) zur visuellen Unterscheidung und/oder Zuordnung der Orientierungen umfasst.

9. Verfahren nach einem der vorangehenden Ansprüche, **dadurch gekennzeichnet, dass** die Ansteuerung der Patientenliege (8) durch die Steuereinrichtung (13) erst bei Erfüllung einer insbesondere eine Bestätigung durch einen Benutzer anzeigenden Triggerbedingung erfolgt.

10. Verfahren nach Anspruch 9, **dadurch gekennzeichnet, dass** die Triggerbedingung die Betätigung eines, insbesondere redundant ausgeführten, Bedienelements (30, 31) an der Hauptmagneteinheit (2) und/oder an dem Positionsfestlegungsmittel (14, 14') auswertet.

11. Verfahren nach einem der vorangehenden Ansprüche, **dadurch gekennzeichnet, dass** mehrere, insbesondere eine Reihenfolge vorgebende und/oder eine zugeordnete Identifikationsinformation an die Steuereinrichtung (13) sendende, Positionsfestlegungsmittel (14, 14') bei mehreren aufeinanderfolgenden Messungen von Magnetresonanzdaten in unterschiedlichen Untersuchungsbereichen verwendet werden.

12. Verfahren nach einem der vorangehenden Ansprüche, **dadurch gekennzeichnet, dass** das Positionsfestlegungsmittel (14, 14') ein, insbesondere quaderförmiges, Gehäuse (15), in dem der Magnetfeldsensor (16) aufgenommen ist, und/oder maximale Ausdehnung von 1 bis 10 cm und/oder ein Volumen von 1 bis 100 Kubikzentimetern aufweist und/oder mittels eines Befestigungsmittels (20) an der Patientenliege (8) und/oder einem Kleidungsstück und/oder einer Decke und/oder einer Lokalspulenanordnung befestigt wird.

13. Verfahren nach Anspruch 12, **dadurch gekennzeichnet, dass** als das Befestigungsmittel (20) ein Clipmittel und/oder ein Klettmittel verwendet wird.

14. Magnetresonanzeinrichtung (1), aufweisend eine Hauptmagneteinheit (2) mit einer insbesondere zylindrischen Patientenaufnahme (3) und einem ein Hauptmagnetfeld erzeugenden Hauptmagneten (4), eine in die Patientenaufnahme (3) entlang einer Längsrichtung einfahrbare Patientenliege (8), eine Steuereinrichtung (13) und ein auf der Patientenliege (8) und/oder einem auf der Patientenliege (8) gelagerten Patienten (7) frei an einer Markerposition positionierbares Positionsfestlegungsmittel (14, 14') mit einem Magnetfeldsensor (16) und einer Kommunikationseinrichtung (19) zur Übertragung von Sensordaten des Magnetfeldsensors (16) an die Steuereinrichtung (13), wobei die Steuereinrichtung (13) aufweist:
- eine Auswertungseinheit (26) zur Auswertung der Sensordaten des Magnetfeldsensors (16) zur Ermittlung der Markerposition in einem Koordinatensystem (10) der Magnetresonanzeinrichtung (1), in dem auch eine Sichtfeldposition eines Sichtfelds (5) der Magnetresonanzeinrichtung (1) bekannt ist,
- eine Ermittlungseinheit (27) zur Ermittlung einer gewünschten Sollposition des Sichtfelds (5) relativ zu der Markerposition aus der Markerposition zumindest in der Längsrichtung der Patientenliege (8) für eine nachfolgende Messung von Magnetresonanzdaten, und
- eine Steuereinheit (28), welche zur Ansteuerung der Patientenliege (8) unter Verwendung der Markerposition derart eingerichtet ist, dass die gewünschte relative Sollposition des Sichtfelds (5) zu der Markerposition eingenommen wird.

## Claims

1. Method for positioning a patient couch (8) in a patient tunnel (3) of a magnetic resonance facility (1), wherein the in particular cylindrical patient tunnel (3) is defined by a main magnet unit (2) with a main magnet (4) generating a main magnetic field, **characterised in that**
- a position determination means (14, 14') comprising an, in particular three-dimensional, magnetic field sensor (16) is positioned at a marker position on the patient couch (8) or on a patient (7) supported on the patient couch (8),
- the sensor data of the magnetic field sensor (16) is evaluated by means of a control facility (13) for establishing the marker position in a coordinate system (10) of the magnetic resonance facility (1), in which a field of view position of a field of view (5) of the magnetic resonance facility (1) is also known,
- a desired required position of the field of view (5) relative to the marker position for a subsequent measurement of magnetic resonance data is determined by the control facility (13) from the marker position at least in a longitudinal direction of the patient couch (8) corresponding to a direction of movement of the patient couch (8), and
- the patient couch (8) is controlled by the control facility (13), using the marker position and the field of view position, in such a way that the desired required position of the field of view (5) relative to the marker position is set.

2. Method according to claim 1, **characterised in that** the desired location of the field of view (5) as the middle of the field of view (6) at least in the longitudinal direction at the marker position is determined by the control facility (13).

3. Method according to claim 1 or 2, **characterised in that** the position determination means (14, 14') has an optical marker (21) provided on its outer side, corresponding to the position of the magnetic field sensor (16) in the position determination means (14, 14'), for visual description of the current marker position.

4. Method according to one of the preceding claims, **characterised in that** the position determination means (14, 14') further has an energy supply device (18) and/or a wireless communication facility (19), by means of which the sensor data is transferred wirelessly to the control facility (13).

5. Method according to claim 4, **characterised in that** the energy supply device (18) is charged by alternating fields occurring during a measurement of magnetic resonance data in the patient tunnel (3) and/or the sensor data is transmitted automatically on request and/or at regular intervals and/or, in particular when a number of position determination means (14, 14') are used, at randomly chosen time intervals.

6. Method according to one of the preceding claims, **characterised in that** a Hall sensor (17) is used as the magnetic field sensor (16) and/or the magnetic field sensor (16) is assigned an orientation sensor, in particular an acceleration sensor.

7. Method according to one of the preceding claims, **characterised in that** by the control facility (13), using the sensor data of the magnetic field sensor (16) and/or of the orientation sensor, orientation information also describing the orientation of the position determination means (14, 14') is determined and the control facility (13), from the orientation information, by means of an orientation to control information, which relates to a subsequent movement of the patient couch (8) and/or a subsequent measurement of magnetic resonance data, and/or determination information, which describes the determination of the required position from the marker position, assignment specification (25) to be assigned, control information and/or determination information, wherein the control facility (13) controls the magnetic resonance facility (1) in accordance with the control information and/or determines the required position in accordance with the determination information.

8. Method according to claim 7, **characterised in that** the control information relates to a support of the patient (7) and/or to a speed of movement of the patient couch (8) and/or to a local coil arrangement to be used and/or to a recording program to be used and/or to a magnetic resonance sequence to be used, and/or that the position determination means (14, 14') comprises optical markings (22) for visual distinction and/or assignment of the orientations provided on its outer side.

9. Method according to one of the preceding claims, **characterised in that** the activation of the patient couch (8) by the control facility (13) only takes place on fulfilment of a trigger condition, in particular indicating a confirmation by a user.

10. Method according to claim 9, **characterised in that** the trigger condition evaluates the actuation of an, in particular redundantly designed, operating element (30, 31) on the main magnet unit (2) and/or on the position determination means (14, 14').

11. Method according to one of the preceding claims, **characterised in that** identification information specifying a number, in particular a series and/or an assigned position determination means (14, 14') sending to the control facility (13), is used for a number of consecutive measurements of magnetic resonance data in different examination regions.

12. Method according to one of the preceding claims, **characterised in that** the position determination means (14, 14') has an, in particular rectangular cuboid, housing (15) in which the magnetic field sensor (16) is accommodated, and/or has a maximum extent of 1 to 10 cm and/or volume of 1 to 100 cubic centimetres and/or is fastened by means of a fastening means (20) to the patient couch (8) and/or to an item of clothing and/or to a cover and/or to a local coil arrangement.

13. Method according to claim 12, **characterised in that** a clip means and or a Velcro-type means is used as the fastening means (20).

14. Magnetic resonance facility (1), having a main magnet unit (2) with an in particular cylindrical patient tunnel (3) and a main magnet (4) generating a main magnetic field, a patient couch (8) able to be moved into the patient tunnel (3) in a longitudinal direction, a control facility (13) and a patient (7) supported on the patient couch (8) and/or a position determination means (14, 14') with a magnetic field sensor (16) freely supported on the patient couch (8) able to be positioned at a marker position and a communication facility (19) for transmission of sensor data of the magnetic field sensor (16) to the control facility (13), wherein the control facility (13) has:
- an evaluation unit (26) for evaluation of the sensor data of the magnetic field sensor (16) for establishing the marker position in a coordinate system (10) of the magnetic resonance facility (1), in which a field of view position of a field of view (5) of the magnetic resonance facility (1) is also known,
- a determination unit (27) for establishing a desired required position of the field of view (5) relative to the marker position from the marker position at least in the longitudinal direction of the patient couch (8) for a subsequent measurement of magnetic resonance data, and
- a control unit (28), which is configured for activation of the patient couch (8) while using the marker position in such a way that the desired relative required position of the field of view (5) for the marker position is assumed.

## Revendications

1. Procédé de mise en position d'une couchette (8) pour un patient dans un logement (3) pour un patient d'un dispositif (1) de résonance magnétique, dans lequel le logement (3) pour un patient, en particulier cylindrique, est défini par une unité (2) magnétique principale ayant un aimant (4) principal produisant un champ magnétique principal, **caractérisé en ce que**
- l'on met en position, en une position de marqueur sur la couchette (8) pour un patient ou sur un patient (7) couché sur la couchette (8) pour le patient, un moyen (14, 14') de fixation de position, en particulier en trois dimensions, comprenant un capteur (16) de champ magnétique,
- l'on exploite, au moyen d'un dispositif (13) de commande, les données du capteur (16) de champ magnétique pour la détermination de la position du marqueur dans un système (10) de coordonnées du dispositif (1) de résonance magnétique, dans lequel une position d'un champ (5) de vue du dispositif (1) de résonance magnétique est connue également,
- par le dispositif (13) de commande, on détermine à partir de la position du marqueur au moins dans une direction longitudinale de la couchette (8) pour le patient, correspondant à une direction de déplacement de la couchette (8) pour le patient, une position de consigne souhaitée du champ (5) de vue par rapport à la position du marqueur pour une mesure à venir ensuite de données de résonance magnétique, et
- l'on commande, par le dispositif (13) de commande, en utilisant la position du marqueur et la position du champ de vue, la couchette (8) pour le patient, de manière à ce que la position de consigne relative souhaitée du champ (5) de vue par rapport à la position du marqueur soit prise.

2. Procédé suivant la revendication 1, **caractérisé en ce que** l'on détermine, par le dispositif (13) de commande, à la position du marqueur au moins dans la direction longitudinale, la position souhaitée du champ (5) de vue comme milieu (6) du champ de vue.

3. Procédé suivant la revendication 1 ou 2, **caractérisé en ce que** le moyen (14, 14') de fixation de position a, pour la description visuelle de la position en cours du marqueur, un marqueur (21) optique prévu sur son côté extérieur correspondant à la position du capteur (16) de champ magnétique dans le moyen (14, 14') de fixation de position.

4. Procédé suivant l'une des revendications précédentes, **caractérisé en ce que** le moyen (14, 14') de fixation de position a en outre un dispositif (18) d'alimentation en énergie et/ou un dispositif (19) de communication sans fil, au moyen duquel on transmet sans fil les données du capteur au dispositif (13) de commande.

5. Procédé suivant la revendication 4, **caractérisé en ce que** l'on charge dans le logement (3) pour le patient le dispositif (18) d'alimentation en énergie par des champs alternatifs se produisant lors d'une mesure de données de champ magnétique et/ou on transmet automatiquement les données de capteur sur demande et/ou d'une manière régulière et/ou, en particulier lors de l'utilisation de plusieurs moyens (14, 14') de fixation de position, à des intervalles de temps choisis au hasard.

6. Procédé suivant l'une des revendications précédentes, **caractérisé en ce que** l'on utilise comme capteur (16) de champ magnétique, un capteur (17) de Hall et/ou un capteur d'orientation, en particulier un capteur d'accélération est affecté au capteur (16) de champ magnétique.

7. Procédé suivant l'une des revendications précédentes, **caractérisé en ce que**, par le dispositif (13) de commande, on détermine, en utilisant les données de capteur du capteur (16) de champ magnétique et/ou du capteur d'orientation, également une information d'orientation décrivant l'orientation du moyen (14, 14') de fixation de position et le dispositif (13) de commande détermine une information de commande et/ou une information de détermination à partir d'une règle (25) d'affectation affectée à des orientations par rapport à des informations de commande, qui concernent un déplacement venant ensuite de la couchette (8) pour le patient et/ou une mesure venant ensuite de données de référence magnétique et/ou d'informations de détermination, qui décrivent la détermination de la position de consigne à partir de la position du marqueur, dans lequel le dispositif (13) de commande commande le dispositif (1) de résonance magnétique suivant l'information de commande et/ou détermine la position de consigne suivant l'information de détermination.

8. Procédé suivant la revendication 7, **caractérisé en ce que** les informations de commande concernent une position du patient (7) et/ou une vitesse de déplacement de la couchette (8) pour le patient et/ou un agencement de bobines local à utiliser et/ou un programme d'enregistrement à utiliser et/ou une séquence de résonance magnétique à utiliser et/ou **en ce que** le moyen (14, 14') de fixation de position comprend des marquages (22) optiques prévus sur son côté extérieur pour distinguer visuellement et/ou affecter les orientations.

9. Procédé suivant l'une des revendications précédentes, **caractérisé en ce que** la commande de la couchette (8) pour le patient s'effectue par le dispositif (13) de commande seulement lorsque est satisfaite une condition de déclenchement indiquant, en particulier, une confirmation par un utilisateur.

10. Procédé suivant la revendication 9, **caractérisé en ce que** la condition de déclenchement exploite l'actionnement d'un élément (30, 31) de service réalisé, en particulier de manière redondante, sur l'unité (2) magnétique principale et/ou sur le moyen (14, 14') de fixation de position.

11. Procédé suivant l'une des revendications précédentes, **caractérisé en ce que** l'on utilise, dans plusieurs mesures successives de données de résonance magnétique, dans des parties à examiner différentes, plusieurs moyens (14, 14') de fixation de position prescrivant en particulier une séquence et/ou envoyant une information d'identification affectée au dispositif (13) de commande.

12. Procédé suivant l'une des revendications précédentes, **caractérisé en ce que** le moyen (14, 14') de fixation de position a un boîtier (15), en particulier parallélépipédique, dans lequel le capteur (16) de champ magnétique est reçu et/ou une étendue maximum de 1 à 10 cm et/ou un volume de 1 à 100 centimètres cube et/ou est fixé, à l'aide d'un moyen (20) de fixation, à la couchette (8) pour le patient et/ou à un vêtement et/ou à une couverture et/ou à un agencement de bobines local.

13. Procédé suivant la revendication 12, **caractérisé en ce que** l'on utilise comme moyen (20) de fixation un moyen à clip et/ou un moyen auto-agrippant.

14. Dispositif (1) de résonance magnétique, comportant une unité (2) ayant un logement (3) pour un patient, en particulier cylindrique, et un aimant (4) principal produisant un champ magnétique principal, une couchette (8) pour un patient pouvant entrer dans le logement (3) pour un patient suivant une direction longitudinale, un dispositif (13) de commande et un moyen (14, 14') de fixation de position pouvant être mis en position librement en une position de marqueur sur la couchette (8) pour le patient et/ou sur un patient (7) couché sur la couchette (8) pour le patient, comprenant un capteur (16) de champ magnétique et un dispositif (19) de communication pour la transmission de données du capteur (16) de champ magnétique au dispositif (13) de commande, dans lequel le dispositif (13) de commande a :
- une unité (26) d'exploitation pour l'exploitation des données du capteur (16) de champ magnétique afin de déterminer la position du marqueur dans un système (10) de coordonnées du dispositif (1) de résonance magnétique, dans lequel une position d'un champ (5) de vue du dispositif (1) de résonance magnétique est également connue,
- une unité (27) de détermination pour la détermination d'une position de consigne souhaitée du champ (5) de vue par rapport à la position du marqueur à partir de la position du marqueur au moins dans la direction longitudinale de la couchette (8) pour un patient pour une mesure à venir ensuite de données de résonance magnétique, et
- une unité (28) de commande, qui est agencée pour la commande de la couchette (8) pour le patient en utilisant la position du marqueur, de manière à ce que la position de consigne relative souhaitée du champ (5) de vue par rapport à la position du marqueur soit prise.
